# EUROPEAN PATENT APPLICATION

(11) **EP 4 062 938 A1**
(43) Date of publication of application: **28.09.2022**
(21) Application number: 20889943.5
(22) Date of filing: 17.11.2020
(51) Int. Cl.: A61K 45/06, A61P 35/00, A61P 35/02, A61P 43/00, A61K 31/437, A61K 31/44, A61K 31/47, A61K 31/506, A61K 31/517, A61K 31/519, A61K 31/5377

(54) **COMBINATION DRUG**

(30) Priority: 18.11.2019 JP 2019208149
(71) Applicant: CHUGAI SEIYAKU KABUSHIKI KAISHA, Tokyo 115-8543 (JP)
(72) Inventor: FUJIMURA Takaaki, Kamakura-shi, Kanagawa 247-8530 (JP); FURUGAKI Koh, Kamakura-shi, Kanagawa 247-8530 (JP); YOSHIMURA Yasushi, Kamakura-shi, Kanagawa 247-8530 (JP); HARADA Naoki, Kamakura-shi, Kanagawa 247-8530 (JP)
(74) Representative: Vossius & Partner Patentanwälte Rechtsanwälte mbB
(86) International application number: PCT/JP2020/042696
(87) International publication number: WO 2021/100677

(57) **Abstract**

By combining a compound having RET kinase inhibitory activity with a CDK4/6 inhibitor, the present inventors have found a drug, a combination, a pharmaceutical composition, and a preparation which are effective for treating or preventing cancer, a method for treating or preventing cancer, or a method for suppressing tumor growth.

## Description

### Technical Field

The present invention relates to a drug, a combination, a pharmaceutical composition, or a preparation which are useful for the treatment or prevention of cancer, and comprise a compound having RET kinase inhibitory activity in combination with a cyclin dependent kinase 4 and/or cyclin dependent kinase 6 (CDK4/6) inhibitor, as well as a method, a product, and the like for treating or preventing cancer.

### Background Art

Rearranged during transfection (RET) is a proto-oncogene of a receptor tyrosine kinase identified in 1985 (Non Patent Literature 1).

It has been reported that genetic abnormalities (point mutations or translocations) in RET result in the production of abnormal kinases, which are involved in canceration (Non Patent Literature 2). For example, it has been reported that, in lung cancer, RET is fused to the kinesin family protein KIF5B and Coiled-Coil Domain Containing 6 (CCDC6) through chromosomal translocation, and generates KIF5B-RET and CCDC6-RET which have active tyrosine kinase activity, thereby gaining oncogenic potential (Non Patent Literatures 3 and 4). In addition, the generation of abnormal kinases due to point mutations such as the cysteine 634 of RET and translocations with the H4 gene and the like has also been reported in thyroid cancer (Non Patent Literatures 5 and 6). It has been reported that compounds having RET kinase inhibitory activity are useful for cancers with these genetic abnormalities (Non Patent Literatures 7 and 8).

Alecensa (generic name: alectinib hydrochloride, Alectinib) is approved in Japan and overseas as an ALK inhibitor, but it has also been reported to have RET kinase inhibitory activity and has been shown in non-clinical studies to exhibit an anti-tumor effect on lung cancer cells positive for the CCDC6-RET fusion gene (Non Patent Literatures 9 and 10).

Cyclin dependent kinase 4 and/or 6 (CDK4/6) are cell cycle promoters and are involved in the initiation and progression of various malignant tumors. CDK4/6 inhibitors are known to suppress the phosphorylation of Retinoblastoma protein (Rb), which regulates the cell cycle, thereby inducing G1 phase arrest and suppressing the growth of cancer cells (Non Patent Literatures 11 to 13).

Examples of CDK4/6 inhibitors approved in Japan and overseas include Ibrance (generic name: Palbociclib), Kisqali (generic name: Ribociclib), and Verzenio (generic name: Abemaciclib).

Palbociclib is approved in Japan for use in combination with endocrine therapeutic agents for hormone receptor-positive, HER2-negative inoperable or recurrent breast cancer, but is not currently approved for use in combination with molecular targeted drugs. However, there have been several reports on the effects of combining CDK4/6 inhibitors with other molecular targeted therapeutic drugs in non-clinical studies (Patent Literature 1). For example, in a PDX model in which HER2-positive breast cancer cells were transplanted, it was shown that Rb and S6RP were strongly suppressed by the combination with EGFR family kinase inhibitors, and that a high anti-tumor effect was exhibited (Non Patent Literature 14). It has also been reported that the combination with an ALK inhibitor suppressed tumor growth in SCID mice transplanted with neuroblastoma cells having ALK gene abnormality by strongly inducing cell cycle arrest and caspase-independent cell death (Patent Literature 2 and Non Patent Literature 15).

However, there have been no reports on the combination of CDK4/6 inhibitors with compounds having RET kinase inhibitory activity.

### Citation List

### Patent Literature

Patent Literature 1: Japanese Patent No. 5832647
Patent Literature 2: Japanese Patent No. 6479812

### Non Patent Literature

Non Patent Literature 1: Cell. 1985 Sep; 42(2): 581-588
Non Patent Literature 2: J Biol Chem. 1997 Apr 4; 272(14): 9043-9047
Non Patent Literature 3: Genome Res. 2012 Mar; 22(3): 436-445
Non Patent Literature 4: Nat Med. 2012 Feb 12; 18(3): 378-381
Non Patent Literature 5: Crit Rev Clin Lab Sci. 2016 Aug; 53(4): 217-227
Non Patent Literature 6: PLoS One. 2016 Nov 1; 11(11): e0165596
Non Patent Literature 7: Cancer. 2016 Dec 15; 122(24): 3856-3864
Non Patent Literature 8: Lancet Respir Med. 2017 Jan; 5(1): 42-50
Non Patent Literature 9: Mol Cancer Ther. 2014; 13: 2910-2918
Non Patent Literature 10: 2016 Nov; 11(11): 2027-2032
Non Patent Literature 11: Mol Cancer Ther. 2004 Nov; 3(11): 1427-1438
Non Patent Literature 12: Oncotarget. 2017 Jul 4; 8(27): 43678-43691
Non Patent Literature 13: Clin Cancer Res. 2014 Jul 15; 20(14): 3763-3774
Non Patent Literature 14: Cancer Cell. 2016 March 14; 29(3): 255-269
Non Patent Literature 15: Clin Cancer Res. 2017 Jun 1; 23(11): 2856-2868

### Summary of Invention

Thus, there is a demand for the development of a drug that is useful for the prevention and treatment of various types of cancers and that has a better drug efficacy than the conventional prophylactic and therapeutic drugs for cancer.

The present invention aims to provide a novel drug, combination, pharmaceutical composition, or preparation comprising a combination of a plurality of drugs, for use in the treatment and prevention of various types of cancer and in prolonging progression-free survival, as well as a method, a product, and the like for treating cancer using the same.

As a result of using a compound having RET kinase inhibitory activity in combination with a CDK4/6 inhibitor, the present inventors unexpectedly found that the combination of these agents exhibit a superior anti-tumor effect than the administration of a single agent, and completed the present invention.

That is, the present invention relates to the following invention.
<1A> A drug for treating or preventing cancer, comprising a compound having RET kinase inhibitory activity in combination with a cyclin dependent kinase 4 and/or cyclin dependent kinase 6 (CDK4/6) inhibitor.
<1B> A drug for treating or preventing cancer, comprising a compound having RET kinase inhibitory activity in combination with a cyclin dependent kinase 4 and/or cyclin dependent kinase 6 (CDK4/6) inhibitor, separately or together.
<1C> A combination of a compound having RET kinase inhibitory activity and a cyclin dependent kinase 4 and/or cyclin dependent kinase 6 (CDK4/6) inhibitor, for treating or preventing cancer.
<1D> A combination of a compound having RET kinase inhibitory activity and a cyclin dependent kinase 4 and/or cyclin dependent kinase 6 (CDK4/6) inhibitor, administered separately or simultaneously, for treating or preventing cancer.
<1E> A pharmaceutical composition for treating or preventing cancer, comprising a compound having RET kinase inhibitory activity in combination with a cyclin dependent kinase 4 and/or cyclin dependent kinase 6 (CDK4/6) inhibitor.
<1F> A pharmaceutical preparation for treating or preventing cancer, comprising a compound having RET kinase inhibitory activity in combination with a cyclin dependent kinase 4 and/or cyclin dependent kinase 6 (CDK4/6) inhibitor.
<1-2> The drug, combination, pharmaceutical composition, or preparation according to <1A> to <1E>, which is in the form of a compounded drug.
<1-3> The drug, combination, or preparation according to <1A> to <1-2>, wherein the compound having RET kinase inhibitory activity and the CDK4/6 inhibitor are administered separately.
<1-4> The drug, combination, or preparation according to <1A> to <1-2>, wherein the compound having RET kinase inhibitory activity and the CDK4/6 inhibitor are administered simultaneously or sequentially.
<2> A drug or pharmaceutical composition for treating or preventing cancer used in combination with a CDK4/6 inhibitor, comprising a compound having RET kinase inhibitory activity as an active ingredient.
<2-2> The drug or pharmaceutical composition according to <2>, wherein the compound having RET kinase inhibitory activity is administered simultaneously with the CDK4/6 inhibitor.
<2-3> The drug or pharmaceutical composition according to <2> to <2-2>, wherein the compound having RET kinase inhibitory activity is administered before or after the administration of the CDK4/6 inhibitor.
<3> A drug or pharmaceutical composition for treating or preventing cancer used in combination with a compound having RET kinase inhibitory activity, comprising a CDK4/6 inhibitor as an active ingredient.
<3-2> The drug or pharmaceutical composition according to <3>, wherein the CDK4/6 inhibitor is administered simultaneously with the compound having RET kinase inhibitory activity.
<3-3> The drug or pharmaceutical composition according to <3> to <3-2>, wherein the CDK4/6 inhibitor is administered before or after the administration of the compound having RET kinase inhibitory activity.
<4> The drug, combination, pharmaceutical composition, or pharmaceutical preparation according to any one of <1A> to <3-3>, wherein the compound having RET kinase inhibitory activity is a compound selected from the group consisting of alectinib, vandetanib, cabozantinib, sorafenib, and selpercatinib, or a salt or hydrate thereof.
<4-2> The drug, combination, pharmaceutical composition, or pharmaceutical preparation according to any one of <1A> to <3-3>, wherein the compound having RET kinase inhibitory activity is a compound selected from the group consisting of alectinib, vandetanib, cabozantinib, and selpercatinib, or a salt or hydrate thereof.
<4-3> The drug, combination, pharmaceutical composition, or pharmaceutical preparation according to any one of <1A> to <3-3>, wherein the compound having RET kinase inhibitory activity is alectinib, or selpercatinib, or a salt thereof.
<4-4> The drug, combination, pharmaceutical composition, or pharmaceutical preparation according to any one of <1A> to <3-3>, wherein the compound having RET kinase inhibitory activity is alectinib, or a salt thereof.
<4-5> The drug, combination, pharmaceutical composition, or pharmaceutical preparation according to any one of <1A> to <3-3>, wherein the compound having RET kinase inhibitory activity is a compound selected from the group consisting of alectinib, pralsetinib, vandetanib, cabozantinib, sorafenib, and selpercatinib, or a salt or hydrate thereof.
<4-6> The drug, combination, pharmaceutical composition, or pharmaceutical preparation according to any one of <1A> to <3-3>, wherein the compound having RET kinase inhibitory activity is a compound selected from the group consisting of alectinib, pralsetinib, vandetanib, cabozantinib, and selpercatinib, or a salt or hydrate thereof.
<4-7> The drug, combination, pharmaceutical composition, or pharmaceutical preparation according to any one of <1A> to <3-3>, wherein the compound having RET kinase inhibitory activity is alectinib, pralsetinib, vandetanib, or selpercatinib, or a salt thereof.
<4-8> The drug, combination, pharmaceutical composition, or pharmaceutical preparation according to any one of <1A> to <3-3>, wherein the compound having RET kinase inhibitory activity is alectinib, pralsetinib, or vandetanib, or a salt thereof.
<4-9> The drug, combination, pharmaceutical composition, or pharmaceutical preparation according to any one of <1A> to <3-3>, wherein the compound having RET kinase inhibitory activity is alectinib hydrochloride.
   Note that, for example, <1A> to <3-n> (n is a sub-number integer) means that <1A> to <1F>, <2>, <2-2> to <2-n>, <3>, and <3-2> to <3-n> are included. The same applies hereafter.
<5> The drug, combination, pharmaceutical composition, or pharmaceutical preparation according to <1A-E> and <1-2> to <4-9>, wherein the alectinib or the salt thereof is administered twice daily at 20 mg, 40 mg, 60 mg, 80 mg, 120 mg, 160 mg, 220 mg, 240 mg, 300 mg, 460 mg, 600 mg, 760 mg, or 900 mg in free form per dose.
<5-2> The preparation according to <1A> to <4-9>, comprising 20 mg, 40 mg, or 150 mg of alectinib or a salt thereof in free form per unit dosage form of the preparation.
<6> The drug, combination, pharmaceutical composition, or preparation according to any one of <1A> to <5-2>, wherein the CDK4/6 inhibitor is selected from the group consisting of palbociclib, abemaciclib, ribociclib, vandetanib, and 2-hydroxy-1-[2-[[9-(4-methylcyclohexyl)pyrido[4,5]pyrrolo[1,2-d]pyrimidin-2-yl]amino]-7,8-dihydro-5H-1,6-naphthyridin-6-yl]ethenone.
<6-2> The drug, combination, pharmaceutical composition, or preparation according to any one of <1A> to <5-2>, wherein the CDK4/6 inhibitor is palbociclib or abemaciclib.
<7> The drug, combination, pharmaceutical composition, or preparation according to any one of <1A> to <6-2>, wherein the cancer has a fusion gene between the RET gene and another gene and/or a fusion protein between the RET protein and another protein.
<7-2> The drug, combination, pharmaceutical composition, or preparation according to <7>, wherein the other gene and protein are KIF5B, CCDC6, NCOA4, or TRIM33.
<7-3> The drug, combination, pharmaceutical composition, or preparation according to <7>, wherein the fusion gene between the RET gene and another gene and the fusion protein between the RET protein and another protein include a tyrosine kinase domain of the RET gene or protein and a coiled-coil domain of the other gene or protein.
<8> The drug, combination, pharmaceutical composition, or preparation according to any one of <1A> to <7-3>, wherein the cancer has a mutation in RET.
<8-2> The drug, combination, pharmaceutical composition, or preparation according to <8>, wherein the mutation in RET is a mutation causing the activation of RET tyrosine kinase.
<9> The drug, combination, pharmaceutical composition, or preparation according to any one of <1A> to <8-2>, wherein the cancer is selected from the group consisting of acute myelocytic leukemia, chronic myelocytic leukemia, acute lymphocytic leukemia, chronic lymphocytic leukemia, Hodgkin's lymphoma, non-Hodgkin's lymphoma, brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, renal cancer, pyeloureteral cancer, bladder cancer, uterine cancer, testicular cancer, prostate cancer, and the tumors metastasized from these tumors.
<9-2> The drug, combination, pharmaceutical composition, or preparation according to any one of <1A> to <8-2>, wherein the cancer is selected from the group consisting of thyroid cancer, lung cancer, bowel cancer, malignant melanoma, and chronic myelocytic leukemia.
<9-3> The drug, combination, pharmaceutical composition, or preparation according to any one of <1A> to <8-2>, wherein the cancer is selected from the group consisting of medullary thyroid cancer, non-small cell lung cancer, bowel cancer, spitzoid neoplasm, and chronic myelomonocytic leukemia.
<10> The drug, combination, pharmaceutical composition, or preparation according to any one of <1A> to <9-3>, wherein the cancer is selected from the group consisting of medullary thyroid cancer, non-small cell lung cancer, and spitzoid neoplasm.
<10-2> The drug, combination, pharmaceutical composition, or preparation according to any one of <1A> to <9-3>, wherein the cancer is medullary thyroid cancer or non-small cell lung cancer.
<10-3> The drug, combination, pharmaceutical composition, or preparation according to any one of <1A> to <9-3>, wherein the cancer is non-small cell lung cancer.
<11> A method for treating or preventing cancer, comprising administering to a subject an effective amount of a compound having RET kinase inhibitory activity in combination with an effective amount of a CDK4/6 inhibitor.
<11-2> The method according to <11>, wherein the compound having RET kinase inhibitory activity and the CDK4/6 inhibitor are administered separately.
<11-3> The method according to <11>, wherein the compound having RET kinase inhibitory activity and the CDK4/6 inhibitor are administered simultaneously or sequentially.
<12> A method for enhancing the efficacy of treatment of cancer with a compound having RET kinase inhibitory activity, comprising administering to a subject an effective amount of a CDK4/6 inhibitor.
<12-2> The method according to <12>, wherein the CDK4/6 inhibitor is administered simultaneously with the compound having RET kinase inhibitory activity.
<12-3> The method according to <12>, wherein the CDK4/6 inhibitor is administered before or after the administration of the compound having RET kinase inhibitory activity.
<13> A method for prolonging the tumor progression-free survival, comprising administering to a subject an effective amount of a compound having RET kinase inhibitory activity in combination with an effective amount of a CDK4/6 inhibitor.
<13-2> The method according to <13>, wherein the compound having RET kinase inhibitory activity and the CDK4/6 inhibitor are administered separately.
<13-3> The method according to <13>, wherein the compound having RET kinase inhibitory activity and the CDK4/6 inhibitor are administered simultaneously or sequentially.
<14> The method according to any one of <12> to <13-3>, wherein the cancer has a fusion gene between the RET gene and another gene and/or a fusion protein between the RET protein and another protein.
<14-2> The method according to <14>, wherein the other gene and protein are KIF5B, CCDC6, NCOA4, or TRIM33.
<14-3> The method according to <14>, wherein the fusion gene between the RET gene and another gene and the fusion protein between the RET protein and another protein include the tyrosine kinase domain of the RET gene or protein and the coiled-coil domain of the other gene or protein.
<15> The method according to any one of <12> to <13-3>, wherein the cancer has a mutation in RET.
<15-2> The method according to <15>, wherein the mutation in RET is a mutation causing the activation of RET tyrosine kinase.
<16> The method according to any one of <12> to <15-2>, wherein the cancer is selected from the group consisting of acute myelocytic leukemia, chronic myelocytic leukemia, acute lymphocytic leukemia, chronic lymphocytic leukemia, Hodgkin's lymphoma, non-Hodgkin's lymphoma, brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, renal cancer, pyeloureteral cancer, bladder cancer, uterine cancer, testicular cancer, prostate cancer, and the tumors metastasized from these tumors.
<16-2> The method according to any one of <12> to <15-2>, wherein the cancer is thyroid cancer, bowel cancer, or lung cancer.
<16-3> The method according to any one of <12> to <15-2>, wherein the cancer is medullary thyroid cancer or non-small cell lung cancer.
<17> The method according to any one of <12> to <16-3>, wherein the cancer is non-small cell lung cancer.
<18> A method for suppressing tumor growth, comprising administering to a subject an effective amount of a compound having RET kinase inhibitory activity in combination with an effective amount of a CDK4/6 inhibitor.
<18-2> The method according to <18>, wherein the compound having RET kinase inhibitory activity and the CDK4/6 inhibitor are administered separately.
<18-3> The method according to <18>, wherein the compound having RET kinase inhibitory activity and the CDK4/6 inhibitor are administered simultaneously or sequentially.
<19> The method according to any one of <18> to <18-3>, wherein the cancer has a fusion gene between the RET gene and another gene and/or a fusion protein between the RET protein and another protein.
<19-1> The method according to <19>, wherein the other gene and protein are KIF5B, CCDC6, NCOA4, or TRIM33.
<19-2> The method according to claim <19>, wherein the fusion gene between the RET gene and another gene and the fusion protein between the RET protein and another protein include the tyrosine kinase domain of the RET gene or protein and the coiled-coil domain of the other gene or protein.
<19-3> The method according to any one of <18> to <18-3>, wherein the cancer has a mutation in RET.
<19-4> The method according to <19-3>, wherein the mutation in RET is a mutation causing the activation of RET tyrosine kinase.
<19-5> The method according to any one of <18> to <18-3>, wherein the cancer is selected from the group consisting of acute myelocytic leukemia, chronic myelocytic leukemia, acute lymphocytic leukemia, chronic lymphocytic leukemia, Hodgkin's lymphoma, non-Hodgkin's lymphoma, brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, renal cancer, pyeloureteral cancer, bladder cancer, uterine cancer, testicular cancer, prostate cancer, and the tumors metastasized from these tumors.
<19-6> The method according to any one of <18> to <18-3>, wherein the cancer is thyroid cancer, bowel cancer, or lung cancer.
<19-7> The method according to any one of <18> to <18-3>, wherein the cancer is medullary thyroid cancer or non-small cell lung cancer.
<19-8> The method according to any one of <18> to <18-3>, wherein the cancer is non-small cell lung cancer.
<20> The method according to any one of <18> to <19-8>, wherein the compound having RET kinase inhibitory activity is a compound selected from the group consisting of alectinib, vandetanib, cabozantinib, sorafenib, and selpercatinib, or a salt or hydrate thereof.
<20-2> The method according to any one of <18> to <19-8>, wherein the compound having RET kinase inhibitory activity is a compound selected from the group consisting of alectinib, vandetanib, cabozantinib, sorafenib, and selpercatinib, or a salt or hydrate thereof.
<20-3> The method according to any one of <18> to <19-8>, wherein the compound having RET kinase inhibitory activity is a compound selected from the group consisting of alectinib and selpercatinib, or a salt thereof.
<20-4> The method according to any one of <18> to <19-8>, wherein the compound having RET kinase inhibitory activity is alectinib, or a salt thereof.
<20-5> The method according to any one of <18> to <19-8>, wherein the compound having RET kinase inhibitory activity is a compound selected from the group consisting of alectinib, pralsetinib, vandetanib, cabozantinib, sorafenib, and selpercatinib, or a salt or hydrate thereof.
<20-6> The method according to any one of <18> to <19-8>, wherein the compound having RET kinase inhibitory activity is a compound selected from the group consisting of alectinib, pralsetinib, vandetanib, cabozantinib, sorafenib, and selpercatinib, or a salt or hydrate thereof.
<20-7> The method according to any one of <18> to <19-8>, wherein the compound having RET kinase inhibitory activity is a compound selected from the group consisting of alectinib, pralsetinib, vandetanib, and selpercatinib, or a salt thereof.
<20-8> The method according to any one of <18> to <19-8>, wherein the compound having RET kinase inhibitory activity is a compound selected from the group consisting of alectinib, pralsetinib, and vandetanib, or a salt thereof.
<20-9> The method according to any one of <18> to <19-8>, wherein the compound having RET kinase inhibitory activity is alectinib hydrochloride.
<21> The method according to any one of <18> to <19-8>, wherein the alectinib or the salt thereof is administered twice daily at 20 mg, 40 mg, 60 mg, 80 mg, 120 mg, 160 mg, 220 mg, 240 mg, 300 mg, 460 mg, 600 mg, 760 mg, or 900 mg in free form per dose.
<22> The method according to any one of <18> to <21>, wherein the CDK4/6 inhibitor is selected from the group consisting of palbociclib, abemaciclib, ribociclib, and 2-hydroxy-1-[2-[[9-(4-methylcyclohexyl)pyrido[4,5]pyrrolo[1,2-d]pyrimidin-2-yl]amino]-7,8-dihydro-5H-1,6-naphthyridin-6-yl]ethenone.
<22-2> The method according to any one of <18> to <21>, wherein the CDK4/6 inhibitor is palbociclib or abemaciclib.
<23> A product comprising: (1) a preparation comprising a compound having RET kinase inhibitory activity, (2) a container, and (3) an instruction or label indicating that the compound having RET kinase inhibitory activity is to be administered to a subject in combination with at least one CDK4/6 inhibitor for treating cancer.
<23-2> A product comprising: (1) a preparation comprising a CDK4/6 inhibitor, (2) a container, and (3) an instruction or label indicating that the CDK4/6 inhibitor is to be administered to a subject in combination with at least one compound having RET kinase inhibitory activity for treating cancer.

### Brief Description of Drawings

[Figure 1] Graph showing the 50% cell growth inhibition concentrations when using alectinib and pemetrexed, paclitaxel, carboplatin, vinorelbine, gemcitabine, irinotecan, palbociclib, SAHA, BKM120, gedatolisib, everolimus, or luminespib, alone and in combination, on human RET fusion gene-positive non-small cell lung cancer cell line LC-2/ad cells.
[Figure 2] Graph showing the 50% cell growth inhibition concentrations when using alectinib and palbociclib or abemaciclib, alone and in combination, on Ba/F3-KIF5B-RET cells, which are murine pro-B cell line Ba/F3 transfected with the KIF5B-RET fusion gene.
[Figure 3] Graph showing the tumor volume (mean + standard deviation) and the rate of change of relative body weight for each administration group when administering alectinib (20 mg/kg, oral administration once daily for 15 days) in combination with palbociclib (75 mg/kg, oral administration once daily for 15 days) to BALB/c-nu (nude) mice transplanted with Ba/F3-KIF5B-RET cells.
[Figure 4] Graph showing the change in Annexin V binding amount when administering alectinib and palbociclib alone and in combination, on LC-2/ad cells and Ba/F3-KIF5B-RET cells.
[Figure 5] Figure showing the expression levels of RET, S6, Rb and their respective phosphoproteins when administering alectinib and palbociclib alone and in combination, on LC-2/ad cells and Ba/F3-KIF5B-RET cells. Actin (ACTB) is used as a loading control.
[Figure 6] Graph showing the 50% cell growth inhibition concentrations when administering BLU-667 or vandetanib, and palbociclib alone and in combination, on LC-2/ad cells and Ba/F3-KIF5B-RET cells.

### Description of Embodiments

The present invention relates to a drug, a combination, a pharmaceutical composition, and a preparation for treating or preventing cancer which is effective for treating cancer, and comprises a compound having RET kinase inhibitory activity in combination with a CDK4/6 inhibitor; a method for treating or preventing cancer; or a method for suppressing tumor growth.

### Compound Having RET Kinase Inhibitory Activity

The "compound having RET kinase inhibitory activity" is also referred to as a RET inhibitor, and means an agent which inhibits the activity of RET kinase. Preferably, it is an agent which binds to RET kinase and has the effect of inhibiting its activity.

Specific examples thereof include the compounds selected from the group consisting of:
- Alectinib (compound name: 9-ethyl-6,6-dimethyl-8-[4-(morpholin-4-yl)-piperidin-l-y1]-11-oxo-6,1 1-dihydro-5H-benzo[b]carbazole-3-carbonitrile) or a salt thereof, preferably alectinib hydrochloride;
- Vandetanib (compound name: N-(4-bromo-2-fluorophenyl)-6-methoxy-7-[(1-methylpiperidin-4-yl)methoxy]quinazolin-4-amine) or a salt thereof;
- BLU-667 (pralsetinib; compound name: cis-N-{(1S)-1-[6-(4-fluoro-1H-pyrazol-1-yl)pyridin-3-yl]ethyl}-1-methoxy-4-{4-methyl-6-[(5-methyl-1H-pyrazol-3-yl)pyrimidin-2-caroimide]) or a salt thereof;
- Cabozantinib (chemical name: N-(4-(6,7-dimethoxyquinolin-4-yloxy)phenyl)-N'-(4-fluorophenyl)cyclopropane-1,1-dicarboxamide) or a salt thereof, preferably cabozantinib (2S)-hydroxybutane-dioate;
- Sorafenib (compound name: 4-{4-[3-(4-chloro-3-trifluoromethylphenyl)ureido]phenoxy}-N²-methylpyridine-2-carboxamide mono(4-methylbenzenesulfonate)) or a salt thereof; and
- Selpercatinib (compound name: 6-(2-hydroxy-2-methylpropoxy)-4-(6-(6-((6-methoxypyridin-3-yl)methyl)-3,6-diazabicyclo(3.1.1)heptan-3-yl)pyridin-3-yl)pyrazolo(1,5-a)pyridine-3-carbonitrile) or a salt thereof.

Preferably, it is alectinib hydrochloride, pralsetinib, vandetanib, cabozantinib (2S)-hydroxybutane-dioate, sorafenib, or selpercatinib.

More preferably, it is alectinib hydrochloride, vandetanib, cabozantinib (2S)-hydroxybutane-dioate, sorafenib, or selpercatinib.

In addition to the above compounds, the following compounds are also known to have RET kinase inhibitory activity (Oncology Review 2018, Vol 12: 352), and therefore can be used in the present claimed invention.

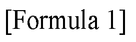

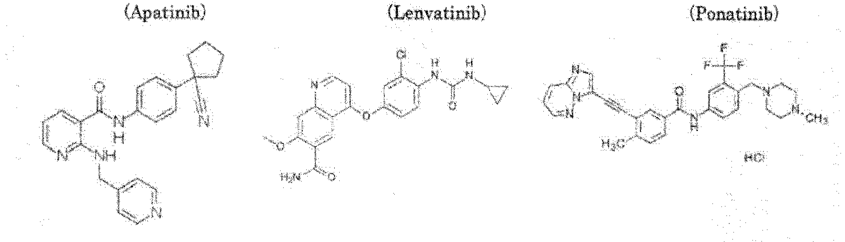

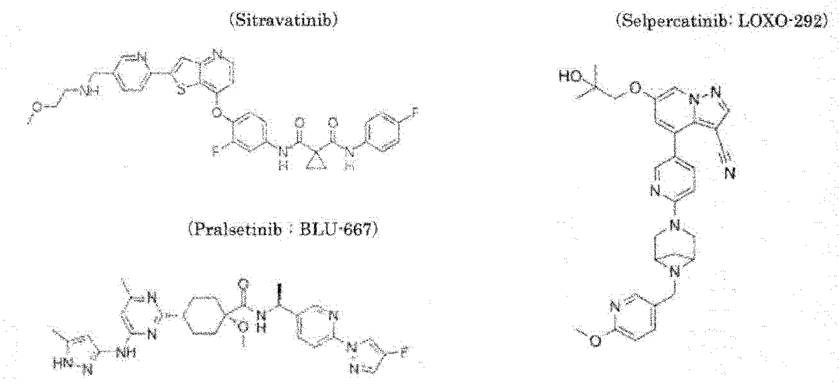

These compounds or the salts thereof are manufactured and sold as pharmaceutical preparations or can be obtained as reagents for research, and alternatively, they can be produced by a publicly known or conventional method. These compounds or the salts thereof also include hydrates, various pharmaceutically acceptable solvates, crystalline polymorphs and the like.

As a route of administration of the compound having RET kinase inhibitory activity used in the present invention, either oral or parenteral administration is suitably used, but preferably, oral administration is suitably used. The dosage form used for oral administration may be appropriately selected from any dosage form such as a liquid, a powder, granules, a tablet, an enteric coated drug, and a capsule. The compound with RET kinase inhibitory activity having such dosage forms is formulated by a method known to those skilled in the art. For example, the compound is formulated by appropriately combining it with a pharmaceutically acceptable carrier or medium, specifically, sterile water or saline, vegetable oil, an emulsifier, a suspending agent, a surfactant, a stabilizer, a flavoring agent, an excipient, a vehicle, a preservative, a binder, and the like, and mixing it in the unit dose form required for generally accepted pharmaceutical practices, then by formulating operations such as freeze-drying and tablet compression.

The compound having RET kinase inhibitory activity may also be used parenterally in the form of an injection, such as a sterile solution or suspension with water or other pharmaceutically acceptable fluids. The amount of active ingredient in these preparations is selected as appropriate so that the appropriate dose within the indicated range may be administered. A sterile composition for injection may be formulated according to normal preparation practices using a vehicle such as distilled water for injection. Examples of aqueous solutions for injection include saline, an isotonic solution containing glucose or other adjuncts, for example, D-sorbitol, D-mannose, D-mannitol, and sodium chloride, which may be appropriately used in combination with an appropriate solubilizing agent, for example, an alcohol, specifically ethanol, a polyalcohol, for example, propylene glycol and polyethylene glycol, and a nonionic surfactant, for example, polysorbate 80 (TM) and HCO-50. Examples of oily liquids include sesame oil and soybean oil, which may be used in combination with benzyl benzoate and benzyl alcohol as a solubilizing agent. In addition, it may also be suitably blended with a buffering agent, for example, a phosphate buffer solution and a sodium acetate buffer solution, a soothing agent, for example, procaine hydrochloride, a stabilizer, for example, benzyl alcohol and phenol, and an antioxidant.

The dose of the compound having RET kinase inhibitory activity according to the present invention may be selected, for example, within the range of 0.0001 mg to 1000 mg per kg of body weight per administration. Alternatively, for example, the dose may be selected within the range of 0.001 mg to 100,000 mg/body per patient. However, the dose of the compound having RET kinase inhibitory activity of the present invention is not limited to these doses.

Examples of a more specific dose of the alectinib, the salt thereof, or the hydrate thereof include 20 mg, 40 mg, 60 mg, 80 mg, 120 mg, 160 mg, 220 mg, 240 mg, 300 mg, 460 mg, 600 mg, 760 mg, and 900 mg in free form per dose, twice daily.

In addition, a more specific dose of pralsetinib, a salt thereof, or a hydrate thereof is 300 mg to 800 mg, preferably 400 mg, in free form once daily. A more specific dose of vandetanib, a salt thereof, or a hydrate thereof is 100 mg to 600 mg, preferably 300 mg, in free form once daily.

The administration period of the compound having the RET kinase inhibitory activity of the present invention is appropriately determined according to the degree of symptoms and side effects, and can be administered until the cancer is treated or the desired therapeutic effect is achieved.

### CDK4/6 Inhibitor

The CDK4/6 inhibitor used in the present invention means a substance capable of directly or indirectly neutralizing, blocking, inhibiting, reducing or interfering with the activity of cyclin dependent kinase 4 (CDK4) or cyclin dependent kinase 6 (CDK6). The substance includes a low molecular weight compound, an antibody, an antisense, a transcription inhibitor, a small interfering RNA (siRNA) and the like.

CDK4 and/or CDK6 are family proteins of the cyclin dependent kinase (CDK), and regulate the initiation, progression, and termination of the cell cycle in mammals. They are important for growth, and dysregulation of this pathway is often observed in breast cancer. CDK4/6 is activated early in the cell cycle by cyclin D1 and other D-type cyclins to promote cell cycle progression through the G1 restriction point (R point). The activated cyclin-CDK complex inactivates the tumor suppressor Rb by phosphorylation and induces the release of the transcriptional activator E2F, which exhibits abnormal cell growth.

Thus, it is publicly known that CDK4/6 inhibitors are used for suppressing the growth of cancer cells, and for example, compounds such as Palbociclib (compound name: 6-acetyl-8-cyclopentyl-5-methyl-2-{[5-(piperazin-1-yl)pyridin-2-yl]amino}pyrido[2,3-d]pyrimidin-7(8H)-one) or a salt thereof;
- Ribociclib (compound name: 7-cyclopentyl-N,N-dimethyl-2-((5-(piperazin-1-yl)pyridin-2-yl)amino)-7H-pyrrolo[2,3-d]pyrimidine) or a salt thereof;
- Abemaciclib (compound name: N-{5-[(4-ethylpiperazin-1-yl)methyl]pyridin-2-yl}-5-fluoro-4-[4-fluoro-2-methyl-1-(1-methylethyl)-1H-benzimidazol-6-yl]pyrimidin-2-amine) or a salt thereof;
   Vandetanib (compound name: N-(4-bromo-2-fluorophenyl)-6-methoxy-7-[(1-methylpiperidin-4-yl)methoxy]quinazolin-4-amine) or a salt thereof;
- Sorafenib (4-{4-[3-(4-chloro-3-trifluoromethylphenyl)ureido]phenoxy}-N2-methylpyridine-2-carboxamide mono(4-methylbenzenesulfonate)) or a salt thereof, preferably sorafenib tosylate;
- AMG-925 (compound name: 2-hydroxy-1-[2-[[9-(4-methylcyclohexyl)pyrido[4,5]pyrrolo[1,2-d]pyrimidin-2-yl]amino]-7,8-dihydro-5H-1,6-naphthyridin-6-yl]ethenone) or a salt thereof;
- Fascaplysin (compound name: 12,13-dihydro-13-oxo-pyrido[1,2-a:3,4-b']diindol-5-ium) or a salt thereof
are used in clinical practice.

Preferably, they are palbociclib, abemaciclib, ribociclib, and AMG-925 or a salt thereof.

These compounds or the salts thereof are manufactured and sold as pharmaceutical preparations or can be obtained as reagents for research, and alternatively, they can be produced by a conventional method. These compounds or the salts thereof also include hydrates, various pharmaceutically acceptable solvates, crystalline polymorphs and the like.

The CDK4/6 inhibitor is formulated according to a conventional method (for example, Remington's Pharmaceutical Science, latest edition, Mack Publishing Company, Easton, U.S.A.) and pharmaceutically acceptable carriers and additives may also be contained. Examples thereof include, but are not limited to, a surfactant, an excipient, a colorant, a flavoring agent, a preservative, a stabilizer, a buffer, a suspending agent, an isotonic agent, a binder, a disintegrant, a lubricant, a flow promoter, and a corrigent, and other commonly used carriers may be used as appropriate. Specifically, suitable examples thereof include trehalose, light anhydrous silicic acid, lactose, crystalline cellulose, mannitol, starch, carmellose calcium, carmellose sodium, hydroxypropyl cellulose, hydroxypropyl methylcellulose, polyvinyl acetal diethylamino acetate, polyvinylpyrrolidone, gelatin, a medium chain triglyceride, polyoxyethylene hydrogenated castor oil 60, refined sugar, carboxymethyl cellulose, corn starch, an inorganic salt, and a polysorbate.

In the present invention, the method for administering the CDK4/6 inhibitor may be carried out by either oral or parenteral administration. Particularly preferred is an administration method by oral administration, and specifically, suitable examples of such administration method include administration by a liquid, a powder, granules, a tablet, an enteric coated drug, a capsule or the like. As an example of administration by injection, the therapeutic drug of the present invention may be administered systemically or locally by intravenous injection, intramuscular injection, intraperitoneal injection, subcutaneous injection, or the like. In addition, the method of administration may be appropriately selected according to the patient's age and symptoms. For example, in the case of an oral agent, the dose may be selected within the range of 0.1 mg/kg to 100 mg/kg, preferably 1 mg/kg to 10 mg/kg per administration. For example, the dose may be selected within the range of 50 mg to 500 mg per patient. However, the dose of the CDK4/6 inhibitor used in the present invention is not limited to these doses.

Depending on the type and severity of the disease, for example, the preferred dosage of palbociclib is within the range of 1 to 5 mg/kg, but not limited thereto, and may be gradually reduced depending on the degree of symptoms and side effects. For the frequency of administration, an administration once a day for a predetermined period of time (for example, 3 weeks) is usually considered as one cycle, followed by a predetermined drug holiday (for example, 1 week), and then the cycle is repeated. The number of cycles varies depending on the type and severity of the disease. The treatment is maintained until the cancer is treated or the desired therapeutic effect is achieved by measuring according to a method known in the art. In one example, palbociclib is administered at a dose of 125 mg once daily for 3 consecutive weeks, and in case side effects or the like are observed, the dose is reduced to 100 mg as a primary dose reduction and to 75 mg as a secondary dose reduction. However, other dosage regimens may also be useful.

The CDK4/6 inhibitor can be administered until the cancer is treated or the desired therapeutic effect is achieved, with the above administration every three weeks considered as one cycle. Specifically, it can be administered over 1 to 36 cycles.

In addition, for example, abemaciclib is administered at a dose of 150 mg once daily, and in case side effects or the like are observed, the dose is reduced to 100 mg as a primary dose reduction and to 50 mg as a secondary dose reduction.

### Drug, Combination, Pharmaceutical Composition, and Method of Treatment and Prevention

One aspect of the present invention is a drug for treating or preventing cancer, comprising a compound having RET kinase inhibitory activity in combination with a CDK4/6 inhibitor.

In the above aspect, "a drug for treating or preventing cancer, comprising a compound having RET kinase inhibitory activity in combination with a CDK4/6 inhibitor" means a drug comprising a compound having RET kinase inhibitory activity in combination with a CDK4/6 inhibitor for simultaneous, separate, or sequential administration in the treatment or prevention of cancer. The drug of the present invention can also be provided in the form of a compounded drug comprising both a compound having RET kinase inhibitory activity and a CDK4/6 inhibitor. In addition, the preparation comprising the compound having RET kinase inhibitory activity and the preparation comprising the CDK4/6 inhibitor may be provided separately, and these preparations may be used simultaneously or sequentially.

In the present invention, simultaneous administration refers to the use of a compound having RET kinase inhibitory activity and a CDK4/6 inhibitor by administering them at the same time, and it may be administered as a compounded drug, administered as a mixture prepared at the time of administration, or preparations of another form may be administered at the same time. When used by simultaneous administration, it may be administered by different routes or it may be administered by the same route, and the dosage forms for administration may be the same or different.

In the present invention, when the compound having RET kinase inhibitory activity and the CDK4/6 inhibitor are administered separately, the order of administration of the compound having RET kinase inhibitory activity and the CDK4/6 inhibitor may be: the compound having RET kinase inhibitory activity is administered after the administration of the CDK4/6 inhibitor; the compound having RET kinase inhibitory activity and the CDK4/6 inhibitor are administered simultaneously; or the CDK4/6 inhibitor is administered after the administration of the compound having RET kinase inhibitory activity.

Sequential administration of the compound having RET kinase inhibitory activity and the CDK4/6 inhibitor according to the present invention means that one agent is administered after the administration of the other. The interval of administration between the compound having RET kinase inhibitory activity and the CDK4/6 inhibitor is not particularly limited and may be set in consideration of factors such as the route of administration and dosage form. For example, the interval of administration between the compound having RET kinase inhibitory activity and the CDK4/6 inhibitor is 0 to 168 hours, preferably 0 to 72 hours, also preferably 0 to 24 hours, and even more preferably 0 to 12 hours. In addition to factors such as the route of administration and dosage form, the respective residual concentrations of the compound having RET kinase inhibitory activity and the CDK4/6 inhibitor in the subject may also be taken into consideration. That is, when the compound having RET kinase inhibitory activity is administered before the administration of the CDK4/6 inhibitor, the CDK4/6 inhibitor may be administered at a time when the residual concentration in the subject of the compound having RET kinase inhibitory activity is detected, such that the desired effect of the CDK4/6 inhibitor is obtained. The concentration may be determined based on the results of separating a sample collected from the subject using a separation device such as various types of chromatography, then analyzing using a method of analysis publicly known to those skilled in the art.

Conversely, when the CDK4/6 inhibitor is administered before the administration of the compound having RET kinase inhibitory activity, the compound having RET kinase inhibitory activity may be administered at a time when the residual concentration in the subject of the CDK4/6 inhibitor is detected, such that the desired effect of the compound having RET kinase inhibitory activity is obtained. The concentration may be determined based on the results of analyzing a sample collected from the subject by an immunoassay such as ELISA publicly known to those skilled in the art.

In the above drug, when the compound having RET kinase inhibitory activity and the CDK4/6 inhibitor are contained and provided in separate preparations, the dosage form of these preparations may be the same or different. For example, both may have a different dosage form than the other, selected from an oral agent, a parenteral agent, an injection, a drip infusion, and an intravenous drip infusion, or both may have the same dosage form selected from an oral agent, a parenteral agent, an injection, a drip infusion, and an intravenous drip infusion. Preferably, the dosage form of both is an oral agent. In addition, the above drug may further be combined with one or more different preparations.

Note that, in the present invention, the pharmaceutical composition comprises the compound having RET kinase inhibitory activity and/or the CDK4/6 inhibitor used in the treatment and/or prevention of the present invention, and may further comprise a pharmaceutically acceptable carrier.

In the present invention, the term "pharmaceutically acceptable carrier" means one or more compatible solid or liquid diluent excipients or encapsulating materials, as previously exemplified, that are suitable for administration to mammals.

In the present invention, a "combination" means a form in which a compound having RET kinase inhibitory activity and a compound that is a CDK4/6 inhibitor can be used separately for the same subject, or a combination of these compounds where each is formulated without mixing them.

In the present invention, the "pharmaceutical preparation", which comprises a compound having RET kinase inhibitory activity in combination with a CDK4/6 inhibitor, includes solid preparations such as tablets, capsules, granules, powders, and pills; liquids such as aqueous and non-aqueous oral solutions and suspensions, and parenteral solutions filled in a container adapted for subdividing into individual doses; lyophilized preparations which can be used by dissolving at the time of use; or preparations combining any of these dosage forms, in which these active ingredients are formulated separately, or comprised in the same preparation. The pharmaceutical preparation comprises, per unit dosage form, 150 mg to 800 mg, preferably 150 mg to 400 mg, and particularly preferably 150 mg to 300 mg of alectinib or a salt thereof in free form. Separate preparations of alectinib or a salt thereof include, specifically, a 150 mg capsule preparation, 150 mg, 300 mg, 600 mg tablets and the like.

In another viewpoint, the present invention provides a drug for treating or preventing cancer used in combination with a CDK4/6 inhibitor, comprising a compound having RET kinase inhibitory activity as an active ingredient. "A drug for treating or preventing cancer used in combination with a CDK4/6 inhibitor, comprising a compound having RET kinase inhibitory activity as an active ingredient" means a drug comprising a compound having RET kinase inhibitory activity as an active ingredient, used for treating or preventing cancer on the condition that it is used in combination with a CDK4/6 inhibitor. When the drug of the present invention comprising a compound having RET kinase inhibitory activity as an active ingredient is used in combination with a CDK4/6 inhibitor, it may be administered simultaneously with the CDK4/6 inhibitor, or it may be administered before or after the administration of the CDK4/6 inhibitor. If a compound having RET kinase inhibitory activity is administered before or after the administration of a CDK4/6 inhibitor, the timing of the administration may be optimized by measuring the residual concentration of the CDK4/6 inhibitor in the subject. The concentration can be determined based on the results of analyzing a sample collected from the subject by an immunoassay such as ELISA described below, which is publicly known to those skilled in the art.

In another viewpoint, the present invention provides a drug for treating or preventing cancer used in combination with a compound having RET kinase inhibitory activity, comprising a CDK4/6 inhibitor as an active ingredient. In the present invention, "a drug for treating or preventing cancer used in combination with a compound having RET kinase inhibitory activity, comprising a CDK4/6 inhibitor as an active ingredient" means a drug comprising a CDK4/6 inhibitor as an active ingredient, used for treating or preventing cancer on the condition that it is used in combination with a compound having RET kinase inhibitory activity. When the drug comprising a CDK4/6 inhibitor as an active ingredient is used in combination with a compound having RET kinase inhibitory activity, it may be administered simultaneously with the compound having RET kinase inhibitory activity, or it may be administered before or after the administration of the compound having RET kinase inhibitory activity. If the CDK4/6 inhibitor is administered before or after the administration of the compound having RET kinase inhibitory activity, the timing of the administration may be optimized by measuring the residual concentration of the compound having RET kinase inhibitory activity in the subject. The concentration can be determined based on the results of separating a sample collected from the subject using a separation device such as various types of chromatography, then analyzing using a method of analysis publicly known to those skilled in the art.

The above invention means that a compound having RET kinase inhibitory activity and a CDK4/6 inhibitor are administered or used (hereinafter, simply referred to as "administered") together, and the order of administration, interval of administration, and the like shall not be construed as limited. The invention may also be used as a product in which a compound having RET kinase inhibitory activity is combined with a CDK4/6 inhibitor. Furthermore, when a compound having RET kinase inhibitory activity is used in combination with a CDK4/6 inhibitor according to the present invention, each may be administered at a dose less than that at which either one is used alone, if desired.

### Cancer Type

The drug of the present invention is useful for the prevention or treatment of diseases such as various cancers such as acute myelocytic leukemia, chronic myelocytic leukemia, acute lymphocytic leukemia, chronic lymphocytic leukemia, Hodgkin's lymphoma, non-Hodgkin's lymphoma, brain tumor, neuroblastoma, glioma, thyroid cancer (for example, medullary thyroid cancer), myelodysplastic syndrome, head and neck cancer, esophageal cancer, stomach cancer, bowel cancer, colorectal cancer, breast cancer, ovarian cancer, lung cancer (for example, non-small cell lung cancer), pancreatic cancer, liver cancer, gallbladder cancer, skin cancer (for example, spitzoid neoplasm), malignant melanoma, renal cancer, pyeloureteral cancer, bladder cancer, uterine cancer, testicular cancer, prostate cancer, and the tumors metastasized from these tumors. Furthermore, the compound of the present invention is useful for the prevention or treatment of the invasion and metastasis of the above cancers.

The drug of the present invention is also useful for the prevention or treatment of cancers with a fusion gene between the RET gene and another gene and/or a fusion protein between the RET protein and another protein. Examples of the "other gene" and "other protein" include KIF5B, CCDC6, NCOA4, TRIM33, CLIP1, and ERC1. Preferably, they are KIF5B, CCDC6, NCOA4 and TRIM33.

In the present invention, the tyrosine kinase domain of the RET gene or protein is the catalytic domain responsible for the reaction of transferring the phosphate group from the γ position of ATP to the tyrosine residue of the protein.

In the present invention, the "coiled-coil domain of the other gene or protein" is the protein-protein interaction domain in which the hydrophobic sites of the α-helix consisting of the heptad FPPFPPP (F: hydrophobic, P: polar amino acid residue) interact with each other.

In particular, the drug of the present invention is useful for the prevention or treatment of cancers caused by tumors having mutations in RET, and/or RET fusion gene-positive cancers. Examples of RET fusion gene-positive cancers include, but are not limited to, thyroid cancer and non-small cell lung cancer.

In the present invention, a tumor having a mutation in RET includes the occurrence of a mutation in the RET gene and/or RET protein which causes the activation of RET tyrosine kinase, or the occurrence of a mutation which activates RET tyrosine kinase and induces canceration (for example, thyroid cancer and lung cancer). The activation of RET tyrosine kinase can be confirmed by detecting phosphorylated RET in tumor tissue by immunostaining with anti-phosphorylated RET antibody and the like.

In the present invention, the activation of RET tyrosine kinase means that amino acid residues contained in RET tyrosine kinase, for example, tyrosine residues, are phosphorylated, and includes an increase (for example, compared to a healthy individual) in the amount of phosphorylated RET tyrosine kinase protein in a subject (for example, a sample collected from a subject). Furthermore, the phosphorylation of a protein targeted by RET tyrosine kinase (hereinafter, target protein) due to the phosphorylation of RET tyrosine kinase is also included. The "activation of RET tyrosine kinase" includes an increase in the amount of phosphorylated RET tyrosine kinase protein, and also of the phosphorylated target protein.

As mutations causing the activation of RET tyrosine kinase, (1) mutations in the cysteine-rich domain of RET, (2) mutations in the tyrosine kinase domain of RET, and (3) the formation of fusion genes between the RET gene and other genes, or of fusion proteins between the RET protein and other proteins have been reported (TRENDS in Genetics, 2006, vol. 22, p. 627-636). The human RET gene is located on chromosome 10 (10q11.2) and consists of 21 exons. The "cysteine-rich domain of RET" refers to the region of RET tyrosine kinase which is rich in cysteine, and the polynucleotides encoding this domain are located on exons 10 and 11. The "tyrosine kinase domain of RET" refers to the region of RET tyrosine kinase having tyrosine kinase activity, and the polynucleotides encoding this domain are located on exons 12 to 18 (TRENDS in Genetics, 2006, vol. 22, p. 627-636).

In the present invention, RET fusion gene-positive means a tumor in which a fusion gene between RET and another gene, such as KIF5B, CCDC6, NCOA4, TRIM33, CLIP1, and ERC1, was detected using a publicly known method, such as a method combining reverse transcription PCR and Sanger sequencing, or in-situ hybridization technology. Specific examples of RET fusion gene-positive cancers include, but are not limited to, those described in WO2014/050781.

In another aspect of the present invention, there is provided a method of enhancing the therapeutic effect of a compound having RET kinase inhibitory activity in the treatment of a cancer patient by the compound having RET kinase inhibitory activity, by using a CDK4/6 inhibitor. Here, enhancing the therapeutic effect means either that the treatment success rate increases; the amount of compound having RET kinase inhibitory activity administered for treatment is reduced; a therapeutic effect is shown in more severe cases; a therapeutic effect is shown in patients with prior treatment failure or exacerbation; the duration of treatment with the compound having RET kinase inhibitor activity is shorter than the expected duration of treatment with the compound alone or the duration of treatment planned before starting the treatment by combined administration, due to the disappearance of the cancer or the like; or that the duration of treatment including the compound having RET kinase inhibitory activity and/or other maintenance therapy is prolonged by the suppression of disease progression. In addition, in another aspect of the present invention, there is provided a method of prolonging the progression-free survival in a subject, comprising administering an effective amount of a compound having RET kinase inhibitory activity in combination with an effective amount of a CDK4/6 inhibitor. In addition, in another aspect of the present invention, there is provided a method of using a compound having RET kinase inhibitory activity or a CDK4/6 inhibitor to produce a pharmaceutical composition for treating or preventing cancer, comprising the compound having RET kinase inhibitory activity and the CDK4/6 inhibitor as active ingredients.

In the present invention, comprising a compound having RET kinase inhibitory activity and/or a CDK4/6 inhibitor as an active ingredient means comprising a compound having RET kinase inhibitory activity and/or a CDK4/6 inhibitor as a main active ingredient, and does not limit the content of the compound having RET kinase inhibitory activity and/or the CDK4/6 inhibitor. In addition, the term "treatment" means that the administration of the drug according to the present invention to a subject causes either the death of cancer cells or a decrease in the number of those cells, the suppression of the growth of cancer cells, the suppression of the metastasis of cancer cells, or the improvement of various symptoms caused by cancer. In addition, the word "prevention" means either preventing an increase in the number of cancer cells that had been reduced from when they grow again, or preventing the regrowth of cancer cells whose growth had been suppressed.

In the present invention, the "effective amount" means the daily dose of each inhibitor when a compound having RET kinase inhibitory activity is administered in combination with a CDK4/6 inhibitor. The dose in the present invention may be the same as the dose when each inhibitor is used alone, or it may be a lower dose than the dose when each inhibitor is used alone. Alternatively, the effective amount in the present invention is the same as the dose when one is used alone, and it may be a lower dose than the dose when the other is used alone.

In the present invention, "progression-free survival (PFS)" refers to the time from treatment (or randomization) to first disease progression or death. In one aspect of the present invention, PFS can be assessed by the Response Evaluation Criteria in Solid Tumors (RECIST). In one aspect of the present invention, PFS can be assessed by CA-125 levels as a determinant of progression.

In the present invention, specific examples of "prolonging progression-free survival" include the use of a compound having RET kinase inhibitory activity in combination with a CDK4/6 inhibitor to prolong PFS compared to the PFS when the compound having RET kinase inhibitory activity or the CDK4/6 inhibitor is administered as a single agent at the same dose used when in combination.

In the present invention, the "subject" means, but is not limited to, a mammal including a human or a non-human mammal, for example, a cow, a horse, a dog, a sheep, or a cat, that is the subject of administration of the drug of the present invention or requires the administration of the drug of the present invention. Preferably, the subject is human. The subject includes a patient (including human and non-human mammal). Specifically, these are patients having, or humans or non-human mammals likely to have, various cancers such as acute myelocytic leukemia, chronic myelocytic leukemia, acute lymphocytic leukemia, chronic lymphocytic leukemia, Hodgkin's lymphoma, non-Hodgkin's lymphoma, brain tumor, neuroblastoma, glioma, thyroid cancer (for example, medullary thyroid cancer), myelodysplastic syndrome, head and neck cancer, esophageal cancer, stomach cancer, bowel cancer, colorectal cancer, breast cancer, ovarian cancer, lung cancer (for example, non-small cell lung cancer), pancreatic cancer, liver cancer, gallbladder cancer, skin cancer (for example, spitzoid neoplasm), malignant melanoma, renal cancer, pyeloureteral cancer, bladder cancer, uterine cancer, testicular cancer, prostate cancer, and the tumors metastasized from these tumors.

### Product

As another aspect of the present invention, a product is provided. In one embodiment, a product is provided for treating or preventing cancer in a subject, and comprises: (a) (1) a preparation comprising a CDK4/6 inhibitor, (2) a container, and (3) an instruction or label indicating that the CDK4/6 inhibitor is to be administered to a subject in combination with at least one compound having RET kinase inhibitory activity for treating cancer in the subject; or (b) (1) a preparation comprising a compound having RET kinase inhibitory activity, (2) a container, and (3) an instruction or label indicating that the compound having RET kinase inhibitory activity is to be administered to a subject in combination with at least one CDK4/6 inhibitor for treating cancer in the subject.

The product comprises a container comprising a preparation comprising a CDK4/6 inhibitor or a compound having RET kinase inhibitory activity.

The product may further comprise a label or instruction on or accompanying the container. In the present invention, the "instruction" means a document normally included in the commercial packaging of a preparation, which includes information on the indications, usage, dose, administration, contraindications and/or warnings regarding the use of the preparation. The "label" means a sheet-shaped article containing the indication of the product name, dosage, dosage form, indications, and the like of a preparation comprising a CDK4/6 inhibitor or a compound having RET kinase inhibitory activity, which is affixed directly to the container.

The label or instruction indicates the indications of the preparation, i.e., that it is to be used for the treatment of cancer and the like. In one embodiment, the label or instruction indicates that the preparation can be used to treat cancer. The label or instruction may also indicate that the preparation can be used to treat other disorders.

Examples of suitable containers include PTP, a bottle, a vial, a syringe, and a blister pack. The container may be formed from a variety of materials such as glass or plastic. These containers may be further packaged in a paper outer box on which the content of the above label and the like are printed.

The use of a compound having RET kinase inhibitory activity in combination with a CDK4/6 inhibitor is expected to reduce the side effects of the agent when administered alone. Here, "side effect" means a clinical, medical, physical, physiological, and/or biochemical effect that is observed and/or measured in a patient receiving treatment for a disease, and that is not part of the intended therapeutic outcome. In general, the effect reduces the health status and/or comfort of the patient being treated, the health risks to the patient being treated, and/or the treatment acceptability to the patient being treated, and makes continuing treatment difficult as it requires appropriate measures such as drug holiday or dose reduction. Specific examples of side effects include myelosuppression (neutropenia, leukopenia, anemia, thrombocytopenia, febrile neutropenia, and lymphopenia), interstitial pneumonia, hepatic dysfunction (elevated ALT, elevated AST, elevated Al-P, and elevated bilirubin), digestive symptoms (diarrhea, nausea, vomiting, stomatitis, elevated lipase, anorexia, elevated amylase, constipation, dyspepsia, dysphagia, abdominal pain, and perforation of the digestive tract), cutaneous symptoms (handfoot syndrome, exfoliative dermatitis, alopecia, rash, skin desquamation, pruritus, skin dryness, flushing, acne, and hypersensitivity reaction), respiratory symptoms (cough, dyspnea, pneumonia, pulmonary infection, and pneumothorax), psychoneurotic symptoms (dysgeusia and dizziness), cardiovascular symptoms (hypertension), fatigue, arthralgia, myalgia, infections (upper respiratory tract infection and urinary tract infection), abnormal laboratory findings (elevated blood creatinine and hypokalemia), thromboembolism, hot flashes, asthenia (muscle weakness), increased tearing, headache, malaise, edema, peripheral edema, weight loss, fever, influenza-like illness, or melalgia. By comparing the frequency, grade, and the like of the side effects observed when used in combination with the frequency, grade, and the like when administered as a single agent, it is possible to confirm whether the side effects are reduced when used in combination.

### Examples

All patents and references expressly cited herein are incorporated herein by reference in their entirety.

Hereinafter, the present invention will be specifically described with reference to the descriptions of Examples, but the present invention is not to be construed as limited by these descriptions.

### Example 1

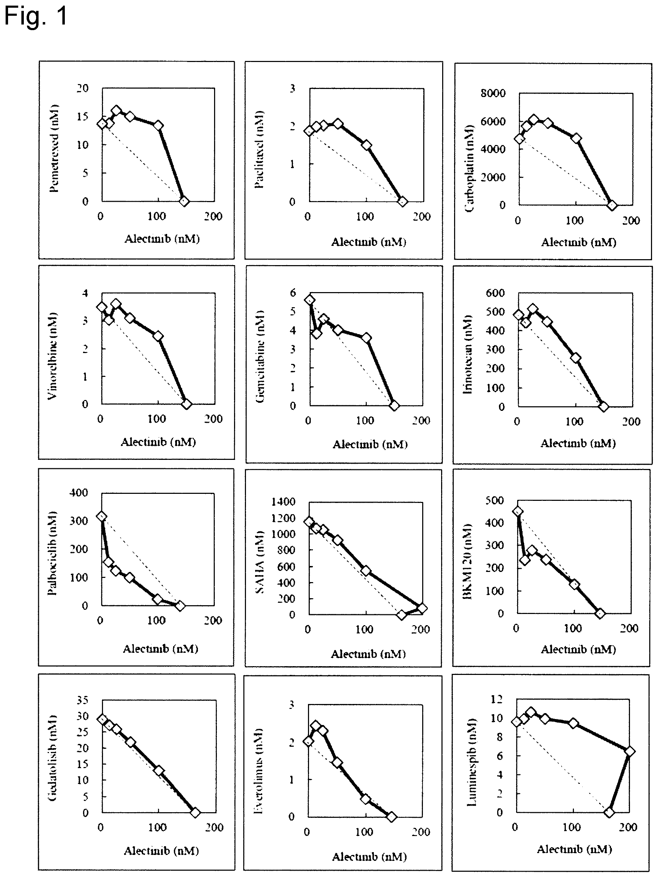

Human RET fusion gene-positive non-small cell lung cancer cell line LC-2/ad cells (RIKEN, J Thorac Oncol. 2012, Dec, 7 (12), 1872-6) were seeded into 96-well plates at 1 × 10⁴ cells per well, and treated with alectinib (synthesized in-house) in combination with each anticancer agent selected from the following 12 compounds of agents used or under clinical development for the treatment of non-small cell lung cancer. The compounds are the folate antimetabolite pemetrexed (FUJIFILM Wako Pure Chemical Corporation), the microtubule inhibitor paclitaxel (FUJIFILM Wako Pure Chemical Corporation), the alkylating agent carboplatin (FUJIFILM Wako Pure Chemical Corporation), the microtubule inhibitor vinorelbine (FUJIFILM Wako Pure Chemical Corporation), the antimetabolite gemcitabine (FUJIFILM Wako Pure Chemical Corporation), the topoisomerase I inhibitor irinotecan (FUJIFILM Wako Pure Chemical Corporation), the CDK4/6 inhibitor palbociclib (Sigma-Aldrich), the HDAC inhibitor SAHA (Tokyo Chemical Industry), the PI3K inhibitor BKM120 (AdooQ BioScience), the PI3K and mTOR inhibitor gedatolisib (Selleck Chemicals LLC), the mTOR inhibitor everolimus (AdooQ BioScience), and the HSP90 inhibitor luminespib (Akt Pharm, Inc.). A cell proliferation assay was conducted after 4 days treatment with each single agent and combination. The concentrations of each agent are shown in Table 1. Figure 1 shows the combination effect analyzed by IC50 isobologram analysis (Pharmacol Res. Perspect. 2015 Jun; 3(3): e00149). Note that, in Figure 1, the horizontal and vertical axes indicate the concentration of alectinib and the combination partner compound, respectively. Here, the point on each axis indicates the 50% cell growth inhibition concentration of each agent used alone, and the dotted line connecting the two points on the axes indicates the additive effect. The data points located below, on, and above the line indicate synergism, additivity, and antagonism, respectively.

As a result, the combination of alectinib with pemetrexed, paclitaxel, carboplatin, vinorelbine, irinotecan, or luminespib showed an antagonistic effect. The combination with gemcitabine, SAHA, gedatolisib, or everolimus showed an additive effect. The combination with BKM120 showed a synergistic effect at some concentrations. The combination with palbociclib showed a synergistic effect as all the points were below the line.

### Example 2

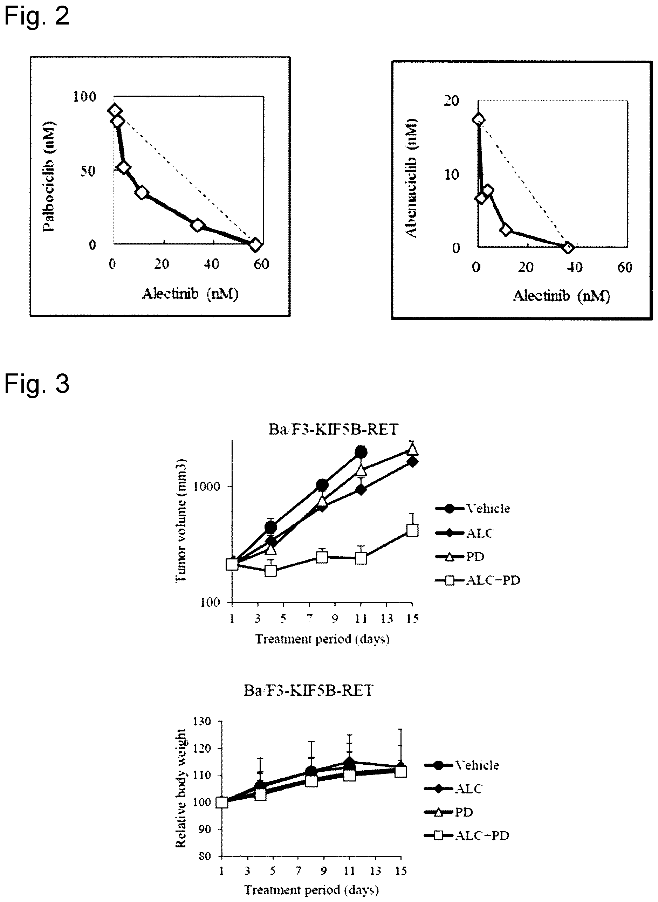

A cell proliferation assay was conducted by seeding Ba/F3-KIF5B-RET cells, which are murine pro-B cell line Ba/F3 transfected with the KIF5B-RET fusion gene (Mol Cancer Ther. 2014; 13: 2910-2918) into 96-well plates at 5 × 10³ cells per well and treating them with alectinib and the CDK4/6 inhibitor palbociclib or the CDK4/6 inhibitor abemaciclib (Selleck Chemicals LLC) in combination or as a single agent for 4 days. The concentrations of each agent are shown in Table 1. Figure 2 shows the combination effect analyzed by IC50 isobologram analysis.

Note that, in Figure 2, the horizontal and vertical axes indicate the concentration of alectinib and palbociclib or abemaciclib, respectively.

As a result, alectinib and palbociclib also showed a synergistic effect in Ba/F3-KIF5B-RET cells. Furthermore, the combination with another CDK4/6 inhibitor abemaciclib also showed a synergistic effect. These results suggest that the combination of alectinib with a CDK4/6 inhibitor has a synergistic effect regardless of the type of CDK4/6 inhibitor.

**[Table 1]**

| Example 1: LC-2/ad cells | |
|---|---|
| Agent Name | Agent Concentration (nM) |
| **Alectinib** | 400, 200, 100, 50.0, 25.0, 12.5 |
| **Pemetrexed** | 300, 150, 75.0, 37.5, 18.8, 9.38, 4.69, 2.34, 1.17 |
| **Paclitaxel** | 10.0, 5.00, 2.50, 1.25, 0.625, 0.313, 0.156, 0.0781, 0.0391 |
| **Carboplatin** | 1.00×10⁴, 6.67×10³, 4.44×10³, 2.96×10³, 1.98×10³, 1.32×10³, 878, 585, 390 |
| **Vinorelbine** | 25.0, 12.5, 6.25, 3.13, 1.56, 0.781, 0.391, 0.195, 0.0977 |
| **Gemcitabine** | 50.0, 25.0, 12.5, 6.25, 3.13, 1.56, 0.781, 0.391, 0.195 |
| **Irinotecan** | 2.50×10³, 1.67×10³, 1.11×10³, 741, 494, 329, 219, 146, 97.5 |
| **Palbociclib** | 2.00×10³, 1.00×10³, 500, 250, 125, 62.5, 31.3, 15.6, 7.81 |
| **SAHA** | 2.00×10⁴, 1.00×10⁴, 5.00×10³, 2.50×10³, 1.25×10³, 625, 313, 156, 78.1 |
| **BKM120** | 2.00×10³, 1.33×10³, 889, 593, 395, 263, 176, 117, 78.0 |
| **Gedatolisib** | 100, 66.7, 44.4, 29.6, 19.8, 13.2, 8.78, 5.85, 3.90 |
| **Everolimus** | 1.00×10⁴, 2.50×10³, 625, 156, 39.1, 9.77, 2.44, 0.610, 0.153 |
| **Luminespib** | 50.0, 33.3, 22.2, 14.8, 9.88, 6.58, 4.39, 2.93, 1.95 |

| Example 2: Ba/F3-KIF5B-RET cells | |
|---|---|
| Agent Name | Agent Concentration (nM) |
| **Alectinib** | 300, 100, 33.3, 11.1, 3.70, 1.23, 0.412, 0.137, 0.0457 |
| **Palbociclib** | 4.00×10³, 1.33×10³, 444, 148, 49.4, 16.5, 5.49, 1.83, 0.610 |
| **Abemaciclib** | 5.00×10³, 1.67×10³, 556, 185, 61.7, 20.6, 6.86, 2.29, 0.762 |

### Example 3

BALB/c-nu (nude) mice (Charles River Laboratories Japan) transplanted with 5 × 10⁶ Ba/F3-KIF5B-RET cells per mouse were treated with alectinib (20 mg/kg, oral administration once daily for 15 days) in combination with palbociclib (75 mg/kg, oral administration once daily for 15 days) eleven days after transplanting the cells. The tumor volume (mean + standard deviation) and the rate of change of relative body weight (mean + standard deviation) for each administration group is shown in Figure 3.

Note that, in Figure 3, the horizontal axis indicates the number of days elapsed when the first day of agent administration is set as 1, and the vertical axis indicates the tumor volume and the rate of change of relative body weight. Vehicle indicates a control group treated with the solvent, ALC indicates a group treated with alectinib only, PD indicates a group treated with palbociclib only, and ALC + PD indicates a group treated with the combination of alectinib and palbociclib.

As a result, the combination administration group showed a higher anti-tumor effect than groups treated with each single agent. On day 11 after the agent administration, the combination administration group showed a statistically significantly higher anti-tumor effect than each single agent administration group and the solvent administration control group. No weight loss was observed in either group compared to the solvent administration group.

### Example 4

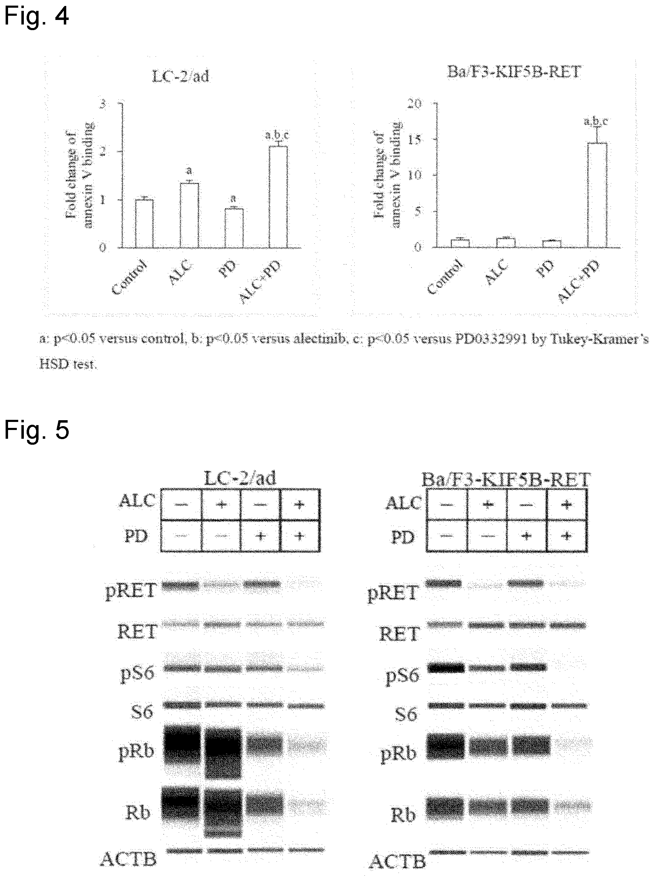

LC-2/ad cells and Ba/F3-KIF5B-RET cells were seeded into 96-well plates at 2 × 10⁴ cells or 1 × 10⁴ cells per well, respectively, and treated with alectinib (100 nM) and palbociclib (100 nM). Three days later, Annexin V binding was observed using the Annexin V detection reagent of the RealTime-Glo Annexin V Apoptosis and Necrosis Assay kit (Promega). The ratio of the fluorescence intensity of each agent treatment group to the control is shown in Figure 4.

Note that, in Figure 4, Control indicates the solvent control group, ALC indicates the alectinib single agent treatment group, PD indicates the palbociclib single agent treatment group, and ALC+PD indicates the alectinib and palbociclib combination treatment group.

As a result, the combination treatment group showed significantly higher Annexin V binding than each single agent groups and solvent control group. This result suggested that these combinations enhanced the induction of apoptosis.

### Example 5

LC-2/ad cells and Ba/F3-KIF5B-RET cells were treated with alectinib (100 nM) and palbociclib (100 nM), and proteins were purified from each cell after 24 hours. Figure 5 shows the results of the expression levels of each protein using the capillary electrophoresis protein analysis system Sally Sue (ProteinSimple).

Note that, in Figure 5, ALC indicates alectinib, PD indicates palbociclib, and - and + indicate agent non-treated and treated, respectively.

As a result, it was confirmed that the combination of both agents suppressed the phosphorylation of S6, which is a protein that promotes the transcription of genes encoding proteins involved in cell cycle progression, compared to the single agents. It was also confirmed that the combination of both agents suppressed the phosphorylation of Rb, which is involved in cell cycle progression, compared to the single agents.

### Example 6

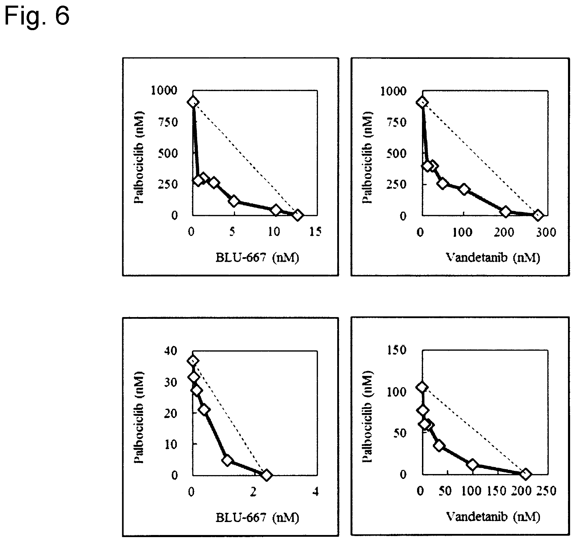

A cell proliferation assay was conducted by seeding LC-2/ad cells and Ba/F3-KIF5B-RET cells into 96-well plates at 1 × 10⁴ cells and 5 × 10³ cells per well, respectively, and treating them with BLU-667 (pralsetinib; Sellck Chemicals LLC) or vandetanib (Cellagen Technology), which are compounds having RET kinase inhibitory activity, and palbociclib, in combination or as a single agent for 4 days. The concentrations of each agent are shown in Table 2. Figure 6 shows the combination effect analyzed by isobologram analysis.

**[Table 2]**

| Example 6: LC-2/ad cells | |
|---|---|
| Agent Name | Agent Concentration (nM) |
| **BLU-667** | 20, 10, 5, 2.5, 1.25, 0.625 |
| **Vandetanib** | 400, 200, 100, 50, 25, 12.5 |
| **Palbociclib** | 2.00×10³, 1.00×10³, 500, 250, 125, 62.5, 31.3, 15.6, 7.81 |

| Example 6: Ba/F3-KIF5B-RET cells | |
|---|---|
| Agent Name | Agent Concentration (nM) |
| **BLU-667** | 10, 3.33, 1.11, 0.37, 0.123, 0.041 |
| **Vandetanib** | 300, 100, 33.3, 11.1, 3.7, 1.23 |
| **Palbociclib** | 4.00×10³, 1.33×10³, 444, 148, 49.4, 16.5, 5.49, 1.83, 0.610 |

Note that, in Figure 6, the horizontal and vertical axes indicate the concentration of BLU-667 or vandetanib and palbociclib, respectively.

As a result, the combination of any compound having RET kinase inhibitory activity and palbociclib also showed a synergistic effect in both cells. These results suggest that the combination of a compound having RET kinase inhibitory activity and a CDK4/6 inhibitor has a synergistic effect regardless of the type of compound having RET kinase inhibitory activity.

### Industrial Applicability

The combination drug of the present claimed invention has a synergistic effect in suppressing cell growth or reducing tumor volume in various RET fusion gene-positive tumor cells and mice transplanted with such cells, and is therefore useful for the prevention or treatment of various cancers with RET mutations.

## Claims

1. A drug for treating or preventing cancer, comprising a compound having RET kinase inhibitory activity in combination with a cyclin dependent kinase 4 and/or cyclin dependent kinase 6 (CDK4/6) inhibitor, separately or together.

2. A drug for treating or preventing cancer used in combination with a CDK4/6 inhibitor, comprising a compound having RET kinase inhibitory activity as an active ingredient.

3. A drug for treating or preventing cancer used in combination with a compound having RET kinase inhibitory activity, comprising a CDK4/6 inhibitor as an active ingredient.

4. The drug according to any one of claims 1 to 3, wherein the compound having RET kinase inhibitory activity is a compound selected from the group consisting of alectinib, pralsetinib, vandetanib, cabozantinib, sorafenib, and selpercatinib, or a salt or hydrate thereof.

5. The drug according to any one of claims 1 to 4, wherein the CDK4/6 inhibitor is selected from the group consisting of palbociclib, abemaciclib, ribociclib, and 2-hydroxy-1-[2-[[9-(4-methylcyclohexyl)pyrido([4,5]pyrrolo[1,2-d])pyrimidin-2-yl]amino]-7,8-dihydro-5H-1,6-naphthyridin-6-yl]ethenone.

6. The drug according to any one of claims 1 to 5, wherein the cancer has a fusion gene between the RET gene and another gene and/or a fusion protein between the RET protein and another protein.

7. The drug according to any one of claims 1 to 6, wherein the cancer has a mutation in RET.

8. The drug according to any one of claims 1 to 7, wherein the cancer is selected from the group consisting of acute myelocytic leukemia, chronic myelocytic leukemia, acute lymphocytic leukemia, chronic lymphocytic leukemia, Hodgkin's lymphoma, non-Hodgkin's lymphoma, brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, renal cancer, pyeloureteral cancer, bladder cancer, uterine cancer, testicular cancer, prostate cancer, and the cancers metastasized from these cancers.

9. A method for treating or preventing cancer, comprising administering to a subject an effective amount of a compound having RET kinase inhibitory activity in combination with an effective amount of a CDK4/6 inhibitor.

10. A method for enhancing the efficacy of treatment of cancer with a compound having RET kinase inhibitory activity, comprising administering to a subject an effective amount of a CDK4/6 inhibitor.

11. A method for enhancing the efficacy of treatment of cancer with a CDK4/6 inhibitor, comprising administering to a subject an effective amount of a compound having RET kinase inhibitory activity.

12. The method according to any one of claims 9 to 11, wherein the cancer has a fusion gene between the RET gene and another gene and/or a fusion protein between the RET protein and another protein.

13. The method according to any one of claims 9 to 12, wherein the cancer has a mutation in RET.

14. The method according to any one of claims 9 to 13, wherein the cancer is selected from the group consisting of acute myelocytic leukemia, chronic myelocytic leukemia, acute lymphocytic leukemia, chronic lymphocytic leukemia, Hodgkin's lymphoma, non-Hodgkin's lymphoma, brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, renal cancer, pyeloureteral cancer, bladder cancer, uterine cancer, testicular cancer, prostate cancer, and the tumors metastasized from these tumors.

15. The method according to any one of claims 9 to 14, wherein the cancer is medullary thyroid cancer, bowel cancer, or non-small cell lung cancer.

16. The method according to any one of claims 9 to 15, wherein the cancer is non-small cell lung cancer.

17. The method according to any one of claims 9 to 16, wherein the compound having RET kinase inhibitory activity is a compound selected from the group consisting of alectinib, pralsetinib, and vandetanib, or a salt thereof.

18. The method according to any one of claims 9 to 17, wherein the compound having RET kinase inhibitory activity is alectinib hydrochloride.

19. The method according to any one of claims 9 to 18, wherein the CDK4/6 inhibitor is palbociclib or abemaciclib.

20. A product comprising: (1) a preparation comprising a compound having RET kinase inhibitory activity, (2) a container, and (3) an instruction or label indicating that the compound having RET kinase inhibitory activity is to be administered to a subject in combination with at least one CDK4/6 inhibitor for treating cancer.
